(19) Europäisches Patentamt
European Patent Office
Office européen des brevets

(11) Numéro de publication : **0 077 720**
**B1**

(12) # FASCICULE DE BREVET EUROPÉEN

(45) Date de publication du fascicule du brevet : **25.07.84**

(21) Numéro de dépôt : **82401870.9**

(22) Date de dépôt : **12.10.82**

(51) Int. Cl.³ : **C 07 C103/365**, C 07 D295/18, C 07 C103/375, C 07 D211/16, C 07 D213/75, A 61 K 31/215, A 61 K 31/44, A 61 K 31/445, A 61 K 31/495

(54) **Dérivés de biphényl alcoyl carboxylates, leur procédé de préparation et leur utilisation comme médicaments.**

(30) Priorité : **14.10.81 FR 8119316**

(43) Date de publication de la demande : **27.04.83 Bulletin 83/17**

(45) Mention de la délivrance du brevet : **25.07.84 Bulletin 84/30**

(84) Etats contractants désignés : **AT BE CH DE GB IT LI LU NL SE**

(56) Documents cités :
**FR-A- 2 185 410**
**FR-M- 5 737**
**FR-M- 6 978**

(73) Titulaire : **PIERRE FABRE S.A.**
**125, rue de la Faisanderie**
**F-75116 Paris (FR)**

(72) Inventeur : **Cousse, Henri**
**La Foun de Los Nobios Chemin de Lastinos**
**F-81100 Castres (FR)**
Inventeur : **Mouzin, Gilbert**
**21, rue Sainte Foy**
**F-81000 Castres (FR)**
Inventeur : **Tarayre, Jean-Pierre**
**Rue des Sports Valdurenque**
**F-81100 Castres (FR)**
Inventeur : **Rieu, Jean-Pierre**
**La Vixère Haute Avenue du Sidobre**
**F-81100 Castres (FR)**

(74) Mandataire : **Corre, Jacques Denis Paul et al**
**Cabinet Regimbeau 26, Avenue Kléber**
**F-75116 Paris (FR)**

**EP 0 077 720 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés de l'acide biphényl acétique, leur procédé de préparation et leur application en thérapeutique.

Ces nouveaux principes actifs possèdent des propriétés anti-inflammatoires et antalgiques et sont utiles notamment comme médicaments pour le traitement des rhumatismes et des algies diverses.

L'invention vise également les compositions pharmaceutiques contenant ces nouveaux composés et leurs sels thérapeutiquement acceptables.

Les composés objet de l'invention répondent à la formule générale (I) :

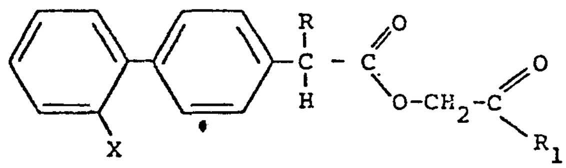

(I)

dans laquelle

X représente un atome d'hydrogène ou d'halogène,

R représente un atome d'hydrogène ou un groupe alcoyle linéaire ou ramifié contenant de 1 à 4 atomes de carbone,

$R_1$ représente les groupes suivants :

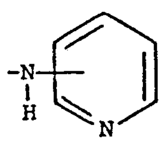 , 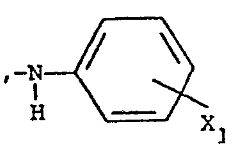 , 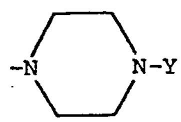 , 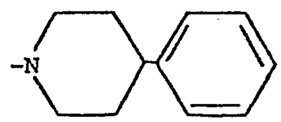

dans lesquels :

$X_1$ représente un atome d'hydrogène ou d'halogène ou un radical $CF_3$,

Y représente un groupe alcoyle linéaire ou ramifié contenant de 1 à 4 atomes de carbone ou un groupe phényl éventuellement substitué par un atome d'halogène ou par un radical $CF_3$.

Dans la présente description le terme « alcoyle » désigne un radical hydrocarboné linéaire ou ramifié contenant de 1 à 4 atomes de carbone.

Les sels thérapeutiquement acceptables des composés de formule générale I sont principalement des sels d'addition ou des acides minéraux ou organiques tels que les acides chlorhydrique, phosphorique, sulfurique, maléique, succinique, fumarique, citrique, etc.

Les nouveaux dérivés d'acide biphényl acétiques de formule générale I selon la présente invention peuvent par exemple être obtenus par traitement d'un acide de formule générale (II) :

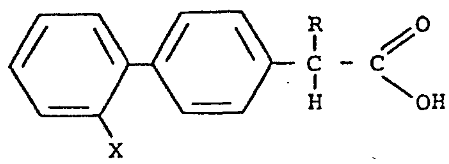

(II)

par un dérivé chloroacétylé de formule générale (III) :

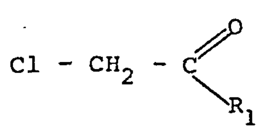

(III)

en présence d'un carbonate alcalin et d'un catalyseur tel que l'iodure de potassium.

On indiquera ci-après à simple titre d'illustration quelques exemples non limitatifs de préparation de dérivés de formule générale I selon la présente invention.

Exemple 1

Préparation du N-phényl, para-biphényl acétoxy acétamide

11 grammes (80 mmoles) de carbonate de potassium sont broyés en présence de 300 mg d'iodure de

potassium ; ce mélange est ajouté à 16,34 g (77 mmoles) d'acide p-biphényl acétique en suspension dans une solution de 11,9 g (70 mmoles) de chloracétanilide dans 200 ml de méthyléthylcétone.

Le mélange réactionnel est porté 5 heures au reflux. Après retour à température ambiante, le mélange est versé dans de l'eau froide. La phase organique est lavée par une solution aqueuse de carbonate de sodium, puis à l'eau jusqu'à neutralité. On sèche sur sulfate de sodium en présence de noir animal, puis on élimine l'insoluble par filtration. Le solvant est évaporé sous pression réduite. Les cristaux obtenus sont recristallisés dans un mélange méthyléthylcétone-hexane (30/70). On récupère après filtration et séchage 73 % de produit de formule :

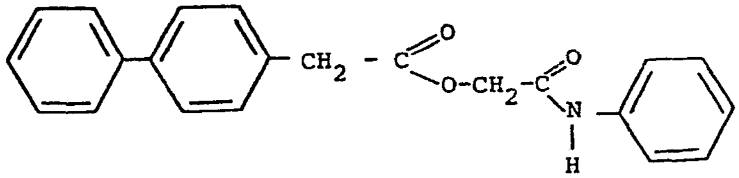

Formule brute : $C_{22}H_{19}NO_3$
Masse moléculaire : 345,4
Cristaux : blancs
Point de fusion : 133 °C
Chromatographie sur plaque :
— support : gel de silice 60 F 254 Merck®
— solvant : acétate d'éthyle-éther de pétrole 30/70
— révélation : UV et iode
— Rf : 0,35.
Spectre infra-rouge (pastille KBr) :
$\nu C = 0$ (ester) 1 750 $cm^{-1}$ ; $\nu C = 0$ (amide) 1 660 $cm^{-1}$.
Spectre RMN (DMSO $d_6$) :

δ ppm : 3,85 (s, 2H, $CH_2$—COO) ; 4,75 (s, 2H, $CO_2$—$CH_2$—$\overset{O}{\overset{\|}{C}}$—N) ; 7-7,8 (m, 14H aromatiques) ; 10,05 (s, 1H, N—H).

## Exemple 2

Préparation du N(méta-trifluorométhyl phényl) para-biphényl acétoxy acétamide

D'une façon similaire à celle décrite dans l'exemple 1, mais en utilisant la méta trifluorométhyl chloracétanilide, on obtient avec un rendement de 55 % le produit de formule :

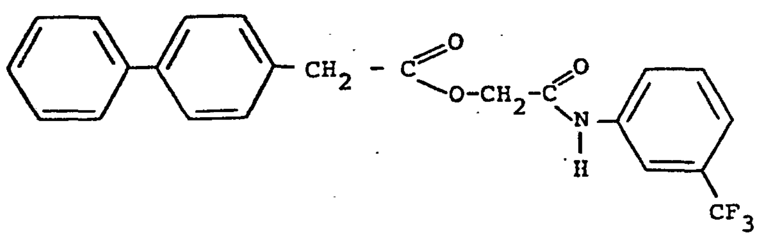

Formule brute : $C_{23}H_{18}F_3NO_3$
Masse moléculaire : 413,4
Cristaux : blancs
Point de fusion : 90 °C
Chromatographie sur plaque :
— support : gel de silice 60 F 254 Merck®
— solvant : acétate d'éthyle-éther de pétrole 30/70
— révélation : UV et iode
— Rf : 0,35
Spectre infra-rouge (KBr) :
$\nu C = 0$ (ester) 1 740 $cm^{-1}$ ; $\nu C = 0$ (amide) 1 670 $cm^{-1}$.
Spectre RMN (DMSO $d_6$) :

δ ppm : 3,9 (s, 2H, $CH_2$—$CO_2$) ; 4,75 (s, 2H, $CO_2$—$CH_2$—$\overset{O}{\overset{\|}{C}}$—N) ; 7,2-7,8 (m, 13H aromatiques) ; 10,4 (s, 1H, NH).

## Exemple 3

Préparation du chlorhydrate de méthyl-4 para-biphényl acétoxy acétyl-1 pipérazine

D'une façon similaire à celle décrite dans l'exemple 1, mais en utilisant la méthyl-4 chloroacétyl-1 pipérazine, on obtient avec un rendement de 60 % le produit de formule :

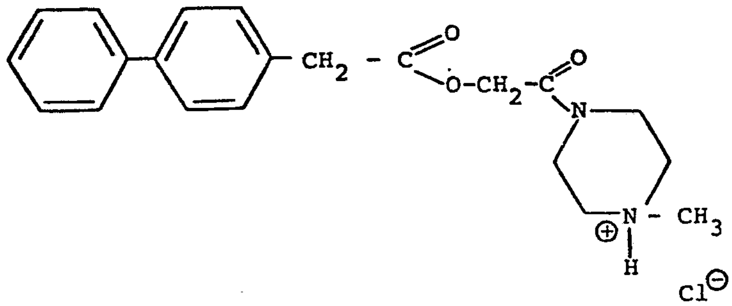

Formule brute : $C_{21}H_{25}ClN_2O_3$
Masse moléculaire : 388,9
Cristaux : blancs
Point de fusion : 174-176 °C
Chromatographie sur plaque :
— support : gel de silice 60 F 254 Merck®
— solvant : chloroforme-méthanol 90/10
— révélation : UV et iode
— Rf : 0,5
Spectre IR (KBr) :
$\nu C = 0$ (ester) 1 745 $cm^{-1}$ ; $\nu C = 0$ (amide) 1 670 $cm^{-1}$.

## Exemple 4

Préparation du phényl-4 para-biphényl acétoxy acétyl-1 pipéridine

D'une manière similaire à celle décrite dans l'exemple 1, mais en utilisant la phényl-4 chloracétyl-1 pipéridine, on obtient avec un rendement de 65 % le produit de formule :

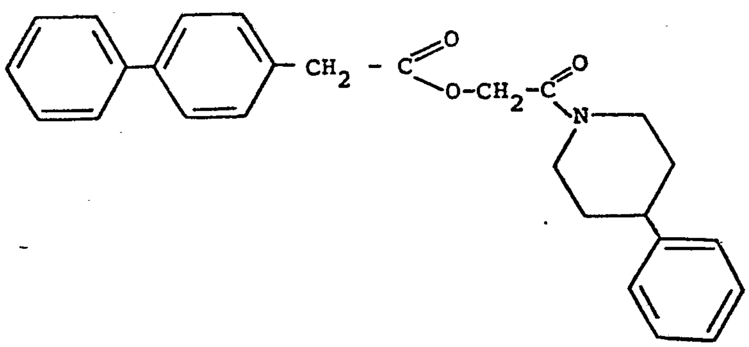

Formule brute : $C_{27}H_{27}NO_3$
Masse moléculaire : 413,5
Cristaux : blancs
Point de fusion : 93 °C
Chromatographie sur plaque :
— support : gel de silice 60 F 254 Merck®
— solvant : acétate d'éthyle-éther de pétrole 50/50
— révélation : UV et iode
— Rf : 0,45
Spectre IR (KBr) :
$\nu C = 0$ (ester) 1 750 $cm^{-1}$ et $\nu C = 0$ (amide) 1 660 $cm^{-1}$.

## Exemple 5

Préparation du chlorhydrate de (méta-trifluorométhyl phényl-4)para-biphénylacétoxy acétyl-1 pipérazine

D'une façon similaire à celle décrite dans l'exemple 1, mais en utilisant la (méta-trifluorométhyl phényl)-4 chloracétyl-1 pipérazine et l'acide chlorhydrique comme agent salifiant, on obtient avec un rendement de 70 % le produit de formule :

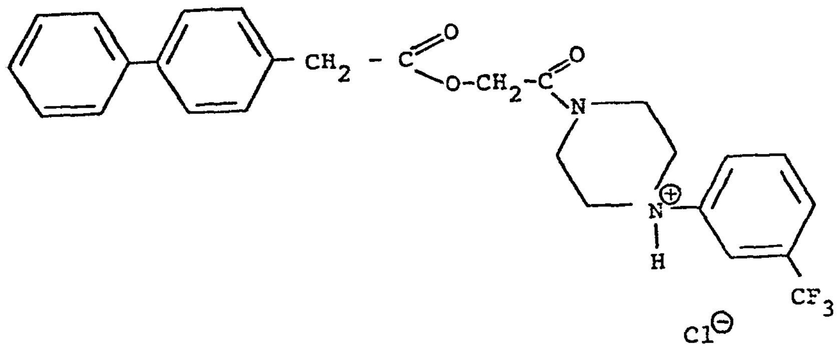

Formule brute : $C_{27}H_{26}ClF_3N_2O_3$
Masse moléculaire : 518,9
Cristaux : blancs
Point de fusion : décomposition lente entre 100 et 110 °C
Chromatographie sur plaque :
— support : gel de silice 60 F 254 Merck®
— solvant : acétate d'éthyle-éther de pétrole
— révélation : UV et iode
— Rf : 0,40
Spectre IR (KBr) :
$\nu C = 0$ (ester) 1 750 $cm^{-1}$ et $\nu C = 0$ (amide) 1 670 $cm^{-1}$.

## Exemple 6

Préparation du phényl-4 [(phényl-4 phényl)acétoxy acétyl]-1 pipérazine

D'une façon similaire à celle décrite dans l'exemple 1, mais en utilisant la phényl-4 chloracétyl-1 pipérazine, on obtient avec un rendement de 71 % le produit de formule :

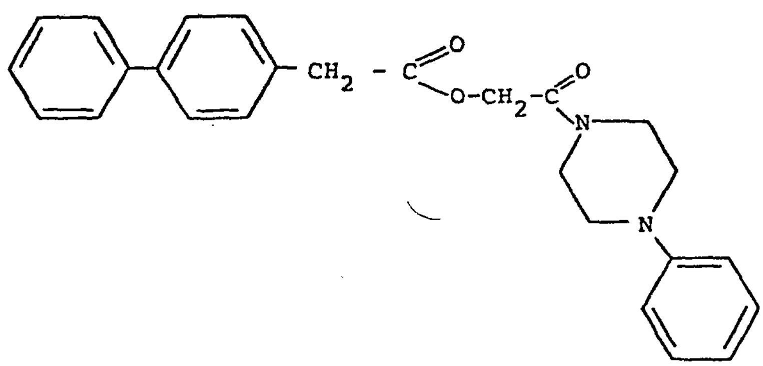

Formule brute : $C_{26}H_{26}N_2O_3$
Masse moléculaire : 414,5
Cristaux : blancs
Point de fusion : 92 °C
Chromatographie sur plaque :
— support : gel de silice 60 F 254 Merck®
— solvant : acétate d'éthyle-éther de pétrole 30/70
— révélation : UV et iode
— Rf : 0,65
Spectre IR (KBr) :
$\nu C = 0$ (ester) 1 740 $cm^{-1}$ et $\nu C = 0$ (amide) 1 650 $cm^{-1}$.
Spectre RMN (DMSO $d_6$) :

δ ppm : 2,9-3,2 (m, 4H, Ph—N—$CH_2$) ; 3,2-3,7 (m, 4H, $\overset{O}{\overset{\|}{C}}$—N—$CH_2$) ; 3,8 (s, 2H, Ph—$CH_2$—CO) ; 4,7 (s, 2H, O—$CH_2$—C=0) ; 6,8-7,7 (m, 14H aromatiques).

Exemple 7

Préparation du chlorhydrate de méthyl-4 [(orthochlorophényl-4 phényl)acétoxy acétyl]-1 pipérazine

D'une façon similaire à celle décrite dans l'exemple 1, mais en utilisant l'acide orthochloro biphényl acétique et la chloracétyl pipérazine, puis en salifiant par l'acide chlorhydrique, on obtient avec un rendement de 83 % le produit de formule :

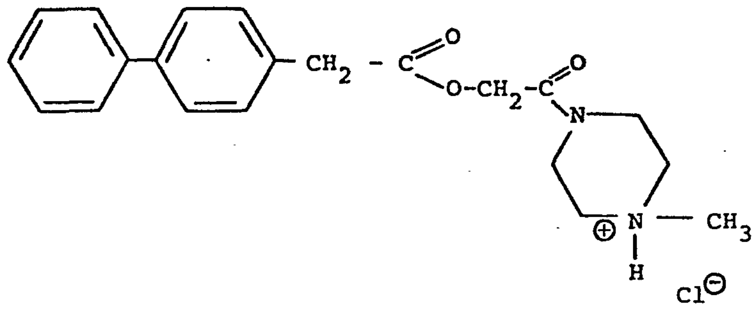

Formule brute : $C_{21}H_{24}Cl_2N_2O_3$
Masse moléculaire : 423,3
Cristaux : blancs
Point de fusion : décomposition lente de 190 à 200 °C
Chromatographie sur plaque :
— support : gel de silice 60 F 254 Merck®
— solvant : chloroforme-méthanol 90/10
— révélation : UV et iode
— Rf : 0,55
Spectre IR (KBr) :
$\nu C = 0$ (ester) 1 750 $cm^{-1}$ et $\nu C = 0$ (amide) 1 670 $cm^{-1}$.

Exemple 8

Chlorhydrate de phényl-4 [(orthochlorophényl)-4 phénylacétoxy acétyl]-1 pipérazine

D'une façon similaire à celle décrite dans l'exemple 1, mais en utilisant l'acide orthochlorobiphényl acétique, et la phényl-4 chloroacétyl pipérazine puis en salifiant par l'acide chlorhydrique, on obtient avec un rendement de 67 % le produit de formule :

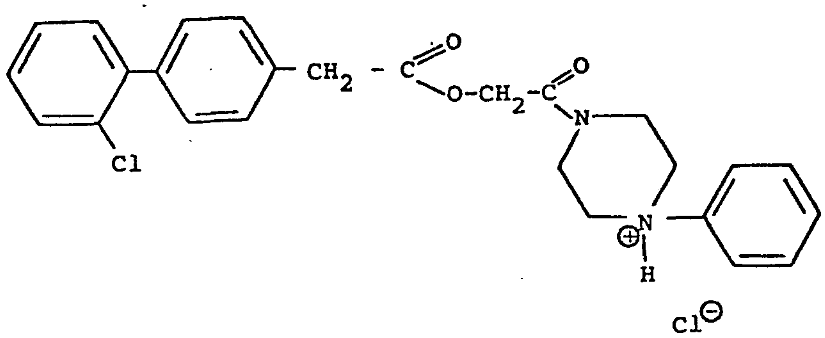

Formule brute : $C_{26}H_{26}Cl_2N_2O_3$
Masse moléculaire : 485,4
Cristaux : blancs
Point de fusion : de 136 à 140 °C
Chromatographie sur plaque :
— support : gel de silice 60 F 254 Merck®
— solvant : chloroforme-méthanol 95/5
— révélation : UV et iode
— Rf : 0,55
Spectre IR (KBr) :
$\nu C = 0$ (ester) 1 750 $cm^{-1}$ et $\nu C = 0$ (amide) 1 670 $cm^{-1}$.

Exemple 9

Préparation du chlorhydrate de méta-tri-fluorométhyl phényl-4 [(orthochlorophényl)-4 phényl acétoxy acétyl]-1 pipérazine

D'une façon similaire à celle décrite dans l'exemple 1, mais en utilisant l'acide orthochloro biphényl acétique et la phényl-4 chloracétyl pipérazine, puis en salifiant avec l'acide chlorhydrique, on obtient avec un rendement de 62 % le produit de formule :

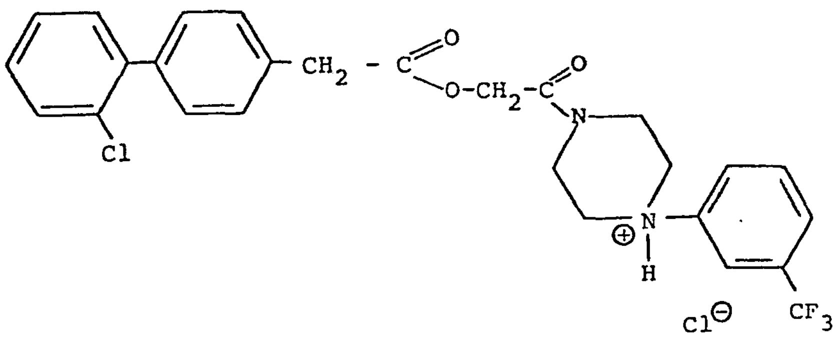

Formule brute : $C_{27}H_{25}Cl_2N_2O_3F_3$
Masse moléculaire : 553,4
Cristaux : blancs
Point de fusion : 134 °C
Chromatographie sur plaque :
— support : gel de silice 60 F 254 Merck®
— solvant : chloroforme-méthanol 95/5
— révélation : UV et iode
— Rf : 0,61
Spectre IR (KBr) :
$\nu C = 0$ (ester) 1 755 $cm^{-1}$ et $\nu C = 0$ (amide) 1 670 $cm^{-1}$.

Exemple 10

Préparation du chlorhydrate de (méta-chlorophényl)-4 [(ortho chlorophényl-4 phényl)acétoxy acétyl]-1 pipérazine

D'une manière similaire à celle décrite dans l'exemple 1, mais en utilisant l'acide orthochloro biphényl acétique et la chloracétyl-1 méta-chlorophényl-4 pipérazine, puis en salifiant avec l'acide chlorhydrique, on obtient avec un rendement de 73 % le produit de formule :

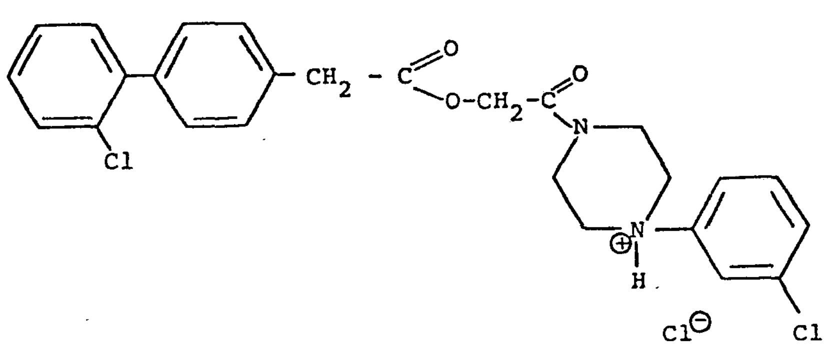

Formule brute : $C_{26}H_{25}Cl_3N_2O_3$
Masse moléculaire : 519,8
Cristaux : blancs
Point de fusion : 128 °C
Chromatographie sur plaque :
— support : gel de silice 60 F 254 Merck®
— solvant : chloroforme-méthanol 95/5
— révélation : UV et iode
— Rf : 0,60

Spectre IR (KBr) :
$\nu C = 0$ (ester) 1 750 $cm^{-1}$ et $\nu C = 0$ (amide) 1 660 $cm^{-1}$.

Exemple 11

Préparation du para-biphényl-2 propionate de [(phényl-4 pipérazinyl-1)-2 oxo-2] éthyle

D'une façon similaire à celle décrite dans l'exemple 1, mais en utilisant l'acide para-biphénylyl-2 propionique et la N'-phényl N-chloracétyl pipérazine, on obtient avec un rendement de 50 % le produit de formule :

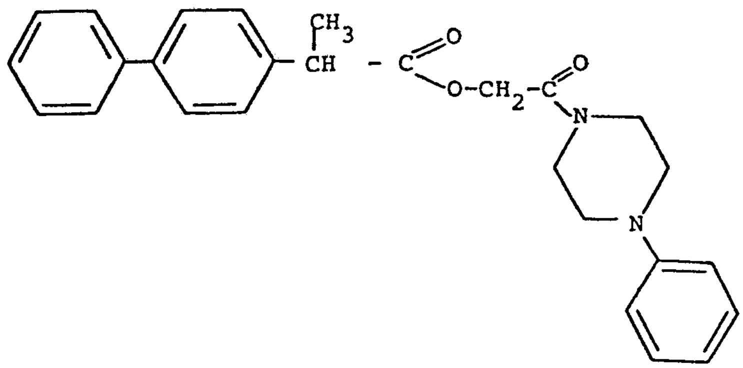

Formule brute : $C_{27}H_{28}N_2O_3$
Masse moléculaire : 428,5
Cristaux : blancs
Point de fusion : 92 °C
Chromatographie sur plaque :
— support : gel de silice 60 F 254 Merck®
— solvant : toluène-acétate d'éthyle 70/30
— révélation : UV et iode
— Rf : 0,38
Spectre IR (KBr) :
$\nu C = 0$ (ester) 1 740 $cm^{-1}$ et $\nu C = 0$ (amide) 1 660 $cm^{-1}$.

Exemple 12

Préparation du chlorhydrate (chloro-2' biphénylyl-4)-2 propionate de (méthyl-4 pipérazino)-2 oxo-2 éthyle

D'une façon similaire à celle décrite dans l'exemple 1, mais en utilisant l'acide (orthochlorophényl-4 phényl)-2 propionique et la méthyl-4 chloroacétyl-1 pipérazine, puis en salifiant par l'acide chlorhydrique, on obtient avec un rendement de 62 % le produit de formule :

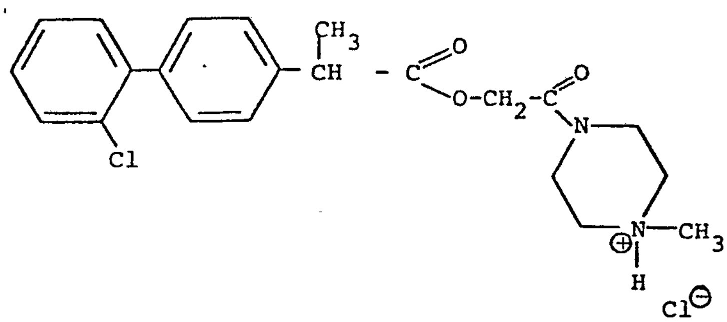

Formule brute : $C_{22}H_{26}Cl_2N_2O_3$
Masse moléculaire : 437,3
Cristaux : blancs
Point de fusion : 210 °C (instantané)
Chromatographie sur plaque :
— support : gel de silice 60 F 254 Merck®
— solvant : chloroforme-méthanol 90/10

— révélation : UV et iode
— Rf : 0,51
Spectre IR (KBr) :
$\nu C = 0$ (ester) 1 745 $cm^{-1}$ et $\nu C = 0$ (amide) 1 675 $cm^{-1}$
Spectre RMN (DMSO $d_6$) :
δ ppm : 1,45 (d, 3H, $CH_3$—C) ; 2,7 (s, 3H, $CH_3$—N) ; 2,8-4,2 (m, 10H, $(CH_2)_4$, échangeable, CH—$CH_3$) ; 4,85 (s, 2H, O—$CH_2$—CO) ; 7,35 (s, 8H aromatiques).

Exemple 13

Préparation du (chloro-2′ biphénylyl-4)-2 propionate de (phényl-4 pipérazino)-2, oxo-2 éthyle

D'une façon similaire à celle décrite dans l'exemple 1, mais en utilisant l'acide (ortho chlorophényl-4 phényl)-2 propionique et la phényl-1 chloracétyl pipérazine, on obtient avec un rendement de 82 % le produit de formule :

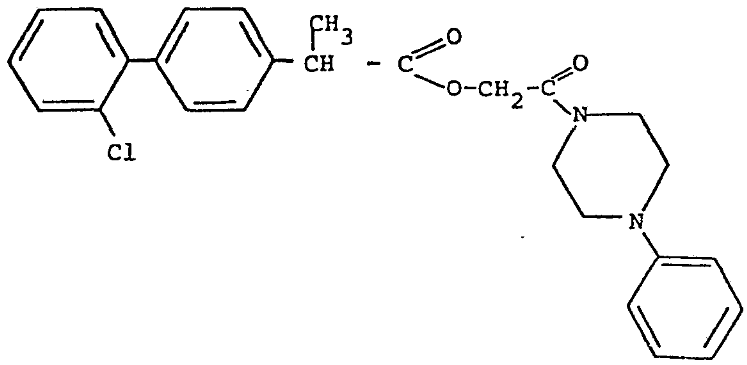

Formule brute : $C_{27}H_{27}ClN_2O_3$
Masse moléculaire : 462,9
Cristaux : beige-clair
Point de fusion : 114 °C
Chromatographie sur plaque :
— support : gel de silice 60 F 254 Merck®
— solvant : chloroforme-méthanol 99/1
— révélation : UV et iode
— Rf : 0,51
Spectre IR (KBr) :
$\nu C = 0$ (ester) 1 745 $cm^{-1}$ et $\nu C = 0$ (amide) 1 650 $cm^{-1}$
Spectre RMN (DMSO $d_6$) :
δ ppm : 2,95 (d, 3H, $CH_3$—C) : 2,8-3,6 (m, 8H, $CH_2$ pipérazine) ; 3,6-4,2 (q, 1H, $CH_3$—CH) ; 4,8 (s, 2H, O—$CH_2$—C=O) ; 6,6-7,5 (m, 13H aromatiques).

Exemple 14

Préparation du (chloro-2′ biphénylyl-4)-2 propionate de (phényl-4 pipéridino)-2 oxo-2 éthyle

D'une manière similaire à celle décrite dans l'exemple 1, mais en utilisant l'acide (orthochlorophényl-4 phényl)-2 propionique et la phényl-4 chloracétyl-1 pipéridine, on obtient avec un rendement de 75 % le produit de formule :

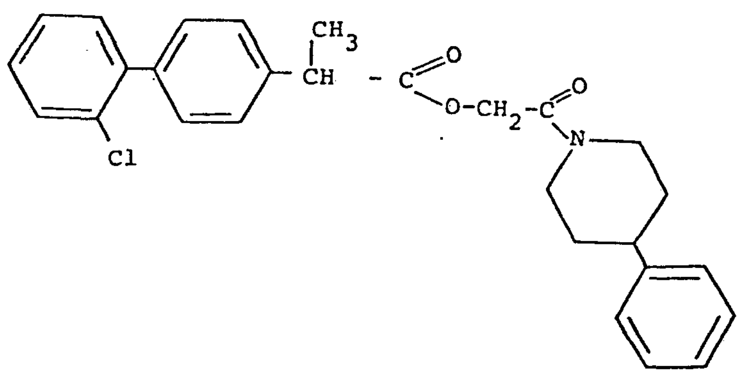

Formule brute : $C_{28}H_{28}ClNO_3$
Masse moléculaire : 461,9
Cristaux : blancs
Point de fusion : 74 °C
Chromatographie sur plaque :
— support : gel de silice 60 F 254 Merck®
— solvant : chloroforme-méthanol 99/1
— révélation : UV et iode
— Rf : 0,35
Spectre IR (KBr) :
$\nu C = 0$ (ester) 1 740 $cm^{-1}$ et $\nu C = 0$ (amide) 1 650 $cm^{-1}$.

Exemple 15

Préparation du (chloro-2' biphénylyl-4)-2 propionate de (amino-2 pyridine)-2 oxo-2 éthyle, oxalate

D'une façon similaire à celle décrite dans l'exemple 1, mais en utilisant l'acide (ortho chloro phényl-4 phényl)-2 propionique et la chloracétyl amino-2 pyridine, on obtient le produit de formule :

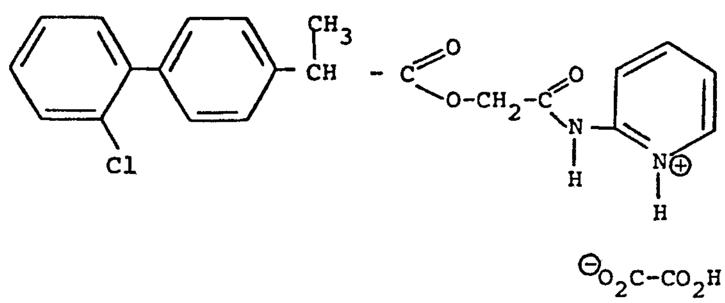

Formule brute : $C_{24}H_{21}ClN_2O_7$
Masse moléculaire : 484,89
Cristaux : beige-clair
Point de fusion : instantané 160 °C
Chromatographie sur plaque :
— support : gel de silice 60 F 254 Merck®
— solvant : chloroforme-acétone 90/10
— révélation : UV et iode
— Rf : 0,81
Spectre IR (KBr) :
$\nu C = 0$ (ester) 1 735 $cm^{-1}$ et $\nu C = 0$ (amide) 1 630 $cm^{-1}$.

Expérimentations

Les dérivés précédemment décrits ont fait l'objet d'essais pharmacologiques et toxicologiques qui ont permis de mettre en évidence d'intéressantes propriétés antalgiques et anti-inflammatoires.

A) Toxicologie

L'étude de la toxicité a été effectuée chez la souris conventionnelle pesant environ 20 grammes.
Les composés de formule générale I selon l'invention ainsi que leurs sels pharmaceutiquement acceptables ont été administrés par voie orale, la $DL_{50}$ a été calculée suivant la méthode de L. C. MILLER et M. L. TAINTER — Proc. Soc. Exper. Biol. Med., 1944, 57, 261. L'ensemble des composés dosés a montré une très faible toxicité et on notera en particulier que les composés des exemples 12 et 14 ont une $DL_{50}$ supérieure à 500 mg/kg.

B) Pharmacologie

L'activité antalgique a été étudiée selon SIEGMUND — J. Pharm. Exptl. Ther., 1957, 119, 453.
Les composés selon la présente invention sont administrés par voie orale 30 minutes avant l'injection de la phényl benzoquinone.
L'ensemble des composés dosés présente une excellente activité antalgique. On mentionnera en particulier que les composés des exemples 12 et 14 ont une $DE_{50}$ respectivement égale à 7 mg/kg et 5 mg/kg.
Les propriétés anti-inflammatoires ont été révélées par le test de l'œdème provoqué par injection de

carragénine dans la patte du rat selon la technique de C. WINTER, E. RIBLEY et G. NUSS — Proc. Soc. Exper. Biol. Med., 1962, 111, 544-547.

Les produits ont été administrés par voie orale en suspension dans le mélange tween-eau 2 heures avant l'expérimentation. Tous les composés dosés présentent une bonne activité anti-inflammatoire. On notera en particulier que le composé de l'exemple 14 présente une $DE_{50}$ égale à 3 mg/kg.

A titre de comparaison, on indiquera dans le tableau ci-après les $DL_{50}$ et $DE_{50}$ obtenues avec quelques-uns des anti-inflammatoires les plus connus.

| Produits | $DL_{50}$ mg/kg p.o. | $DE_{50}$ (carragénine) mg/kg p.o. |
|---|---|---|
| Naproxen | 400 | 5 |
| Ibuprofen | 900 | 14 |
| Aspirine | 1500 | 135 |
| Phénylbutazone | 400 | 30 |
| Indométacine | 20 | 4 |

C) Applications thérapeutiques

Compte tenu de leur parfaite tolérance et de leurs propriétés pharmacologiques, les composés objet de l'invention peuvent être utilisés en thérapeutique humaine ou animale dans le traitement d'algies rebelles à caractères inflammatoires justiciables de traitement prolongé.

Les résultats cliniques se sont avérés satisfaisants dans le cas de rhumatismes inflammatoires ou dégénératifs.

Les préparations pharmaceutiques contenant ces principes actifs peuvent être administrées par voie orale, parentérale ou rectale. La dose par unité de prise est généralement comprise entre 25 et 150 mg. Ces compositions pharmaceutiques peuvent également contenir d'autres principes actifs pharmaceutiquement et thérapeutiquement acceptables.

Quelques exemples de préparations pharmaceutiques contenant des principes actifs objet d'expérimentation sont donnés ci-après à titre indicatif et non limitatif.

a) Comprimés

Chlorhydrate de (chloro-2' biphénylyl-4)-2 propionate de (méthyl-4 pipérazino)-2 oxo-2 éthyle — 50 mg
Excipient : lactose

b) Gélules

(chloro-2' biphénylyl-4)-2 propionate de (phényl-4 pipéridino)-2 oxo-2 éthyle — 25 mg

c) Suppositoires adultes

(chloro-2' biphénylyl-4)-2 propionate de (phényl-4 pipéridino)-2 oxo-2 éthyle — 50 mg
Méprobamate — 100 mg
Glycérides semi-synthétiques q.s.p. 1 suppositoire de 1 g

d) Soluté injectable

Chlorhydrate de (chloro-2' biphénylyl-4)-2 propionate de (méthyl-4 pipérazino)-2 oxo-2 éthyle — 10 mg
Eau pour préparation injectable — 2 ml

**Revendications** (pour les Etats contractants : BE, CH, DE, GB, IT, LI, LU, NL, SE)

1. Dérivés de biphényl alcoyl carboxylates, répondant à la formule générale (I) :

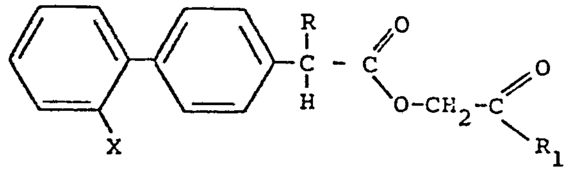

(I)

dans laquelle

X représente un atome d'hydrogène ou d'halogène,

R représente un atome d'hydrogène ou un groupe alcoyle linéaire ou ramifié contenant de 1 à 4 atomes de carbone,

$R_1$ représente les groupes suivants :

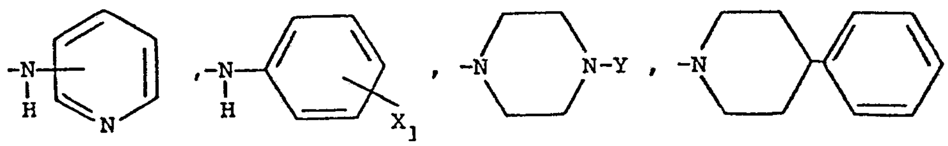

dans lesquels :

$X_1$ représente un atome d'hydrogène ou d'halogène ou un radical $CF_3$,

Y représente un groupe alcoyle linéaire ou ramifié contenant de 1 à 4 atomes de carbone ou un groupe phényl éventuellement substitué par un atome d'halogène ou par un radical $CF_3$,

ainsi que leurs sels thérapeutiquement acceptables.

2. Dérivés de formule générale I selon la revendication 1, caractérisé en ce qu'il est choisi parmi :

— le N-phényl, para-biphényl acétoxy acétamide ;
— le N(méta-trifluorométhyl phényl) para-biphényl acétoxy acétamide ;
— le chlorhydrate de méthyl-4 para-biphényl acétoxy acétyl-1 pipérazine ;
— le phényl-4 para-biphényl acétoxy acétyl-1 pipéridine ;
— le chlorhydrate de (méta-trifluorométhyl phényl-4) para-biphényl acétoxy acétyl-1 pipérazine ;
— la phényl-4 [(phényl-4 phényl) acétoxy acétyl]-1 pipérazine ;
— le chlorhydrate de méthyl-4 [(orthochlorophényl-4 phényl) acétoxy acétyl]-1 pipérazine ;
— le chlorhydrate de phényl-4 [(orthochlorophényl)-4 phénylacétoxy acétyl]-1 pipérazine ;
— le chlorhydrate de méta-tri-fluorométhyl phényl-4 [(orthochlorophényl)-4 phényl acétoxy acétyl]-1 pipérazine ;
— le chlorhydrate de (méta-chlorophényl)-4 [(orthochlorophényl-4 phényl) acétoxy acétyl[-1 pipérazine ;
— le para-biphényl-2 propionate de [(phényl-4 pipérazinyl-1)-2 oxo-2] éthyle ;
— le chlorhydrate (chloro-2′ biphénylyl-4)-2 propionate de (méthyl-4 pipérazino)-2 oxo-2 éthyle ;
— le (chloro-2′ biphénylyl-4)-2 propionate de (phényl-4 pipérazino)-2 oxo-2 éthyle ;
— le (chloro-2′ biphénylyl-4)-2 propionate de (phényl-4 pipéridino)-2 oxo-2 éthyle, et
— le (chloro-2′ biphénylyl-4)-2 propionate de (amino-2 pyridine)-2 oxo-2 éthyle, oxalate.

3. Procédé de préparation des dérivés de formule générale I selon l'une des revendications 1 et 2, caractérisé en ce que l'on traite un acide de formule (II) :

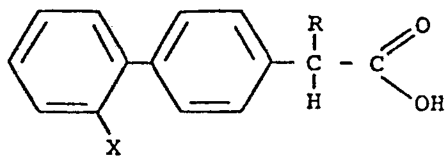

(II)

par un dérivé chloroacétylé de formule générale (III) :

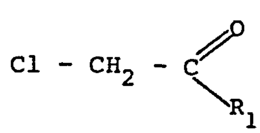

(III)

en présence de carbonate alcalin et d'un catalyseur, les symboles X, R et $R_1$ ayant la même signification que dans la revendication 1.

4. Procédé selon la revendication 3, caractérisé en ce que le carbonate alcalin est le carbone de potassium.

5. Procédé selon l'une des revendications 3 et 4, caractérisé en ce que le catalyseur est l'iodure de potassium.

6. A titre de médicaments nouveaux utiles notamment dans le traitement des algies rebelles et des syndromes inflammatoires douloureux, les produits selon l'une des revendications 1 et 2.

7. Compositions pharmaceutiques caractérisées en ce qu'elles contiennent en tant que principe actif un dérivé de formule générale I selon l'une des revendications 1 et 2.

**Revendications** (pour l'Etat contractant AT)

1. Procédé de préparation de dérivés de biphényl alcoyl carboxylates, répondant à la formule générale (I) :

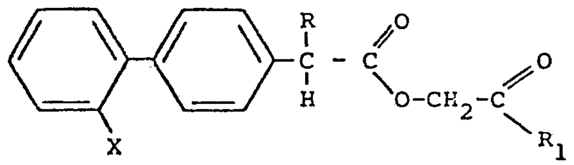
(I)

dans laquelle

X représente un atome d'hydrogène ou d'halogène,

R représente un atome d'hydrogène ou un groupe alcoyle linéaire ou ramifié contenant de 1 à 4 atomes de carbone,

$R_1$ représente les groupes suivants :

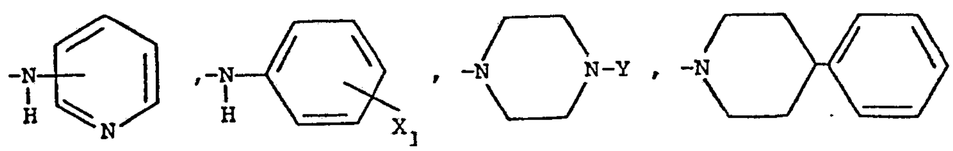

dans lesquels :

$X_1$ représente un atome d'hydrogène ou d'halogène ou un radical $CF_3$,

Y représente un groupe alcoyle linéaire ou ramifié contenant de 1 à 4 atomes de carbone ou un groupe phényl éventuellement substitué par un atome d'halogène ou par un radical $CF_3$,

ainsi que leurs sels thérapeutiquement acceptables, caractérisé en ce que l'on traite un acide de formule (II) :

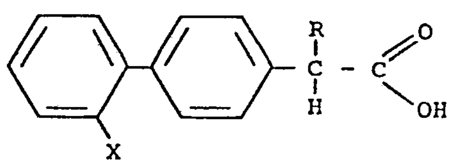
(II)

par un dérivé chloroacétylé de formule générale (III) :

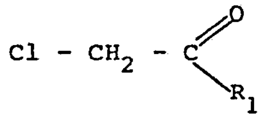
(III)

en présence de carbonate alcalin et d'un catalyseur, les symboles X, R et $R_1$ ayant la même signification que donnée précédemment.

2. Procédé selon la revendication 1, caractérisé en ce que le carbonate alcalin est le carbone de potassium.

3. Procédé selon l'une des revendications 1 et 2, caractérisé en ce que le catalyseur est l'iodure de potassium.

4. Procédé selon l'une des revendications 1 à 3, caractérisé en ce que l'on prépare un dérivé choisi parmi :

— le N-phényl, para-biphényl acétoxy acétamide ;
— le N(méta-trifluorométhyl phényl) para-biphényl acétoxy acétamide ;
— le chlorhydrate de méthyl-4 para-biphényl acétoxy acétyl-1 pipérazine ;
— le phényl-4 para-biphényl acétoxy acétyl-1 pipéridine ;
— le chlorhydrate de (méta-trifluorométhyl phényl-4) para-biphényl acétoxy acétyl-1 pipérazine ;
— la phényl-4 [(phényl-4 phényl) acétoxy acétyl]-1 pipérazine ;
— le chlorhydrate de méthyl-4 [(orthochlorophényl-4 phényl) acétoxy acétyl]-1 pipérazine ;
— le chlorhydrate de phényl-4 [(orthochlorophényl)-4 phénylacétoxy acétyl]-1 pipérazine ;
— le chlorhydrate de méta-tri-fluorométhyl phényl-4 [(orthochlorophényl)-4 phényl acétoxy acétyl]-1 pipérazine ;
— le chlorhydrate de (méta-chlorophényl-4 [(orthochlorophényl-4 phényl) acétoxy acétyl]-1 pipérazine ;
— le para-biphényl-2 propionate de [(phényl-4 pipérazinyl-1)-2 oxo-2] éthyle ;
— le chlorhydrate (chloro-2′ biphénylyl-4)-2 propionate de (méthyl-4 pipérazino)-2 oxo-2 éthyle ;
— le (chloro-2′ biphénylyl-4)-2 propionate de (phényl-4 pipérazino)-2 oxo-2 éthyle ;
— le (chloro-2′ biphénylyl-4)-2 propionate de (phényl-4 pipéridino)-2 oxo-2 éthyle, et
— le (chloro-2′ biphénylyl-4)-2 propionate de (amino-2 pyridine)-2 oxo-2 éthyle, oxalate.

**Claims** (for the Contracting States : BE, CH, DE, GB, IT, LI, LU, NL, SE)

1. Biphenyl alkyl carboxylated derivatives, having the general formula (I) :

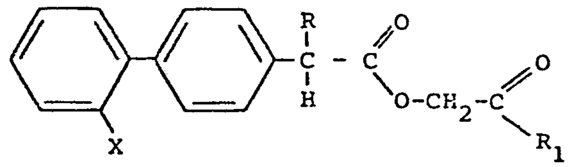

(I)

in which
X represents an atom of hydrogen or of a halogen,
R represents an atom of hydrogen or a linear or branched alkyl group containing 1 to 4 atoms of carbon,
$R_1$ represents the following groups :

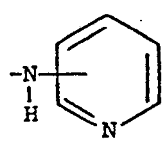 , 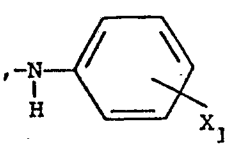 , 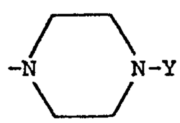 , 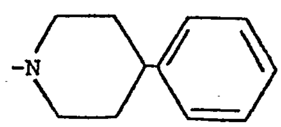

in which :
$X_1$ represents an atom of hydrogen or of a halogen or a $CF_3$ radical,
Y represents a linear or branched alkyl group containing 1 to 4 atoms of carbon or a phenyl group possibly substituted by an atom of a halogen or by a $CF_3$,
as well as their therapeutically acceptable salts.

2. Derivatives of the général formula I according to claim 1, characterised in that it is chosen amongst :

— the N-phenyl, para-biphenyl acetoxy acetamide ;
— the N(meta-trifluoromethyl phenyl) para-biphenyl acetoxy acetamide ;
— the chlorhydrate of 4-methyl, para-biphenyl acetoxy 1-acetyl piperazine ;
— the 4-phenyl para-biphenyl acetoxy 1-acetyl piperidine ;
— the chlorhydrate of (meta-trifluoromethyl 4-phenyl) para-biphenyl acetoxy 1-acetyl piperazine ;
— the 4-phenyl 1-[(4-phenyl phenyl) acetoxy acetyl] piperazine ;
— the chlorhydrate of 4-methyl 1-[(4-orthochlorophenyl phenyl) acetoxy acetyl] piperazine ;
— the chlorhydrate of 4-phenyl 1-[4-(orthochlorophenyl) phenylacetoxy acetyl] piperazine ;
— the chlorhydrate of meta-tri-fluoromethyl 4-phenyl 1-[4-(orthochlorophenyl) phenyl acetoxy acetyl] piperazine ;
— the chlorhydrate of 4-(meta-chlorophenyl) 1-[(4-orthochlorophenyl phenyl) acetoxy acetyl] piperazine ;
— the para-2-biphenyl propionate of 2-[(4-phenyl 1-piperazinyl) 2-oxo] ethyl :
— the chlorhydrate 2-(2′-chloro 4-biphenylyl) propionate of 2-(4-methyl piperazino) 2-oxo ethyl ;
— the 2-(2′-chloro 4-biphenylyl) propionate of 2-(4-phenyl piperazino) 2-oxo ethyl ;

— the 2-(2'-chloro 4-biphenylyl) propionate of 2-(4-phenyl piperidino) 2-oxo ethyl, and
— the 2-(2'-chloro 4-biphenylyl) propionate of 2-(2-amino pyridine) 2-oxo ethyl, oxalate.

3. Process for the preparation of derivatives of the general formula I according to either of claims 1 and 2, characterised in that an acid of the formula (II) :

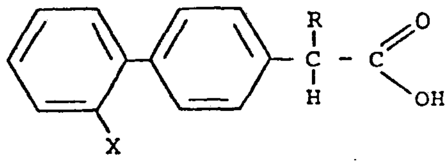

(II)

is treated by a chloracetylated derivative of the general formula (III) :

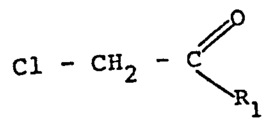

(III)

in the presence of an alkaline carbonate and of a catalyst, the symbols X, R and $R_1$ having the same signification as in claim 1.

4. A process according to claim 3, characterised in that the alkaline carbonate is potassium carbonate.

5. A process according to either of claims 3 and 4, characterised in that the catalyst is potassium iodide.

6. By way of novel medicines particularly useful in the treatment of obstinate fever and painful inflammatory syndromes, the products according to either one of claims 1 and 2.

7. Pharmaceutical compositions characterised in that they contain by way of principal constituent a derivative of the general formula I according to either of claims 1 and 2.

**Claims** (for the Contracting State AT)

1. Process for the preparation of biphenyl alkyl carboxylated derivatives, having the general formula (I) :

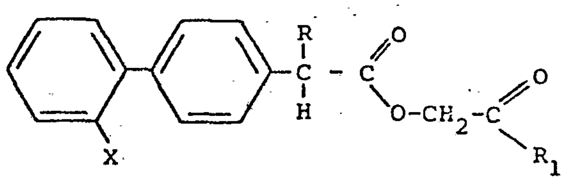

(I)

in which :

X represents an atom of hydrogen or of a halogen,

R represents an atom of hydrogen or a linear or branched alkyl group containing 1 to 4 atoms of carbon,

$R_1$ represents the following groups :

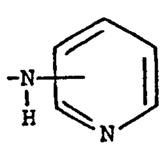 , 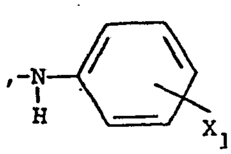 , 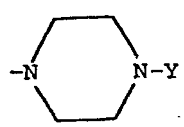 , 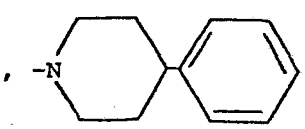

in which :

$X_1$ represents an atom of hydrogen or of a halogen or a $CF_3$ radical,

Y represents a linear or branched alkyl group containing 1 to 4 atoms of carbon or a phenyl group possibly substituted by an atom of a halogen or by a $CF_3$,

as well as their therapeutically acceptable salts, characterised in that an acid of the formula (II) :

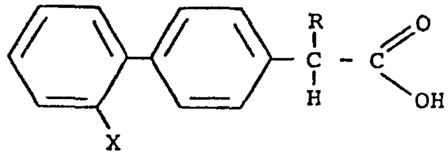

(II)

is treated by a chloracetylated derivative of the general formula (III) :

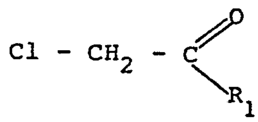

(III)

in the presence of an alkaline carbonate and of a catalyst, the symbols X, R and $R_1$ having the same signification as above.

2. Process according to claim 1, characterised in that the alkaline carbonate is potassium carbonate.

3. Process according to either of claims 1 and 2, characterised in that the catalyst is potassium iodide.

4. Process according to either of claims 1 to 3, characterised in that the following derivatives are prepared :

— the N-phenyl, para-biphenyl acetoxy acetamide ;
— the N(meta-trifluoromethyl phenyl) para-biphenyl acetoxy acetamide ;
— the chlorhydrate of 4-methyl, para-biphenyl acetoxy 1-acetyl piperazine ;
— the 4-phenyl para-biphenyl acetoxy 1-acetyl piperidine ;
— the chlorhydrate of (meta-trifluoromethyl 4-phenyl) para-biphenyl acetoxy 1-acetyl piperazine ;
— the 4-phenyl 1-[(4-phenyl phenyl) acetoxy acetyl] piperazine ;
— the chlorhydrate of 4-methyl 1-[(4-orthochlorophenyl phenyl) acetoxy acetyl] piperazine ;
— the chlorhydrate of 4-phenyl 1-[4-(orthochlorophenyl) phenylacetoxy acetyl] piperazine ;
— the chlorhydrate of meta-tri-fluoromethyl 4-phenyl 1-[4-orthochlorophenyl) phenyl acetoxy acetyl] piperazine ;
— the chlorhydrate of 4-(meta-chlorophenyl) 1-[(4-orthochlorophenyl phenyl) acetoxy acetyl] piperazine ;
— the para-2-biphenyl propionate of 2-[(4-phenyl 1-piperazinyl) 2-oxo] ethyl ;
— the chlorhydrate 2-(2'chloro 4-biphenylyl) propionate of 2(-methyl piperazino) 2-oxo ethyl ;
— the 2-(2'-chloro 4-biphenylyl) propionate of 2-(4-phenyl piperazino) 2-oxo ethyl ;
— the 2-(2'-chloro 4-biphenylyl) propionate of 2-(4-phenyl piperidino) 2-oxo ethyl, and
— the 2-(2'-chloro 4-biphenylyl) propionate of 2-(2-amino pyridine) 2-oxo ethyl, oxalate.

**Ansprüche** (für die Vertragsstaaten : BE, CH, DE, GB, IT, LI, LU, NL, SE)

1. Derivate von Biphenylalkylcarboxylaten entsprechend der allgemeinen Formel (I) :

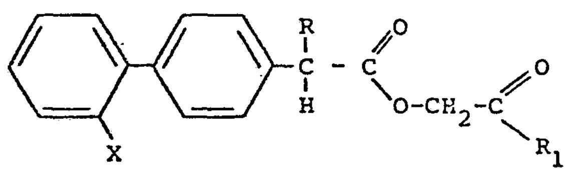

(I)

in welcher :

X ein Wasserstoffatom oder ein Halogenatom darstellt,

R ein Wasserstoffatom oder eine geradkettige oder verzweigte Alkylgruppe mit 1 bis 4 Kohlenstoffatomen darstellt,

$R_1$ eine der nachstehenden Gruppen darstellt :

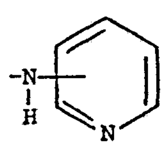 , 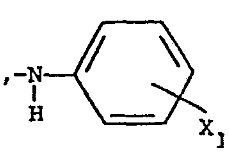 , 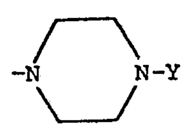 , 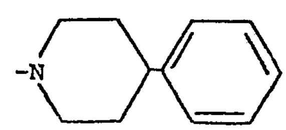

in welchen :

$X_1$ ein Wasserstoffatom oder ein Halogenatom oder einen $CF_3$-Rest darstellt,

Y eine geradkettige oder verzweigte Alkylgruppe mit 1 bis 4 Kohlenstoffatomen oder eine Phenylgruppe darstellt, die gegebenenfalls durch ein Halogenatom oder durch einen $CF_3$-Rest substituiert ist, sowie die therapeutisch akzeptablen Salze dieser Derivate.

2. Derivate der allgemeinen Formel (I) nach Anspruch 1, dadurch gekennzeichnet, daß sie zu den folgenden Verbindungen gehören :

— N-Phenyl-para-biphenylacetoxyacetamid ;
— N(meta-Trifluormethylphenyl)-para-biphenyl-acetoxyacetamid ;
— Chlorhydrat von Methyl-4-para-biphenylacetoxy-acetyl-1-piperazin ;
— Phenyl-4-para-biphenylacetoxyacetyl-1-piperidin ;
— Chlorhydrat von (meta-Trifluormethylphenyl-4)-para-biphenylacetoxyacetyl-1-piperazin ;
— Phenyl-4-[(phenyl-4-phenyl)acetoxyacetyl]-1-piperazin ;
— Chlorhydrat von Methyl-4-[(orthochlorophenyl-4-phenyl) acetoxyacetyl)]-1-piperazin ;
— Chlorhydrat von Phenyl-4-[(orthochlorophenyl)-4-phenylacetoxyacetyl)]-1-piperazin ;
— Chlorhydrat von meta-Trifluoromethylphenyl-4-[(orthochlorophenyl)-4-phenylacetoxyacetyl)]-1-piperazin ;
— Chlorhydrat von (meta-Chlorophenyl)-4-[(orthochlorophenyl-4-phenyl)acetoxyacetyl)]-1-piperazin ;
— para-Biphenyl-2-propionat von [(Phenyl-4-piperazinyl-1)-2-oxo-2]äthyl ;
— Chlorhydrat-(chloro-2'-biphenyl-4)-2-propionat von (Methyl-4-piperazino)-2-oxc-2-äthyl ;
— (Chloro-2'-biphenylyl-4)-2-propionat von (Phenyl-4-piperazino)-2-oxo-2-äthyl ;
— (Chloro-2'-biphenylyl-4)-2-propionat von (Phenyl-4-piperidino)-2-oxo-2-äthyl und
— (Chloro-2'-biphenylyl-4)-2-propionat von (Amino-2-pyridin)-2-oxo-2-äthyl-Oxalat.

3. Verfahren zur Herstellung von Derivaten der allgemeinen Formel (I) gemäß einem der Ansprüche 1 und 2, dadurch gekennzeichnet, daß man eine Säure der Formel (II) :

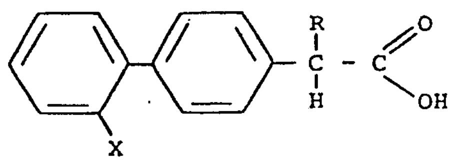

(II)

mit einem Chloroacetylderivat der allgemeinen Formel (III) :

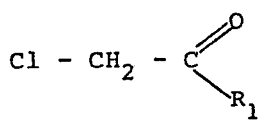

(III)

wobei die Symbole X, R und $R_1$ die gleiche Bedeutung wie in Anspruch 1 haben, in Gegenwart eines Alkalicarbonats und eines Katalysators behandelt.

4. Verfahren nach Anspruch 3, dadurch gekennzeichnet, daß als Alkalicarbonat Kaliumcarbonat verwendet wird.

5. Verfahren nach einem der Ansprüche 3 und 4, dadurch gekennzeichnet, daß als Katalysator Kaliumjodid verwendet wird.

6. Die Produkte gemäß einem der Ansprüche 1 und 2 als Arzneimittel, insbesondere zur Behandlung von hartnäckigen Algien und schmerzhaften Entzündungssyndromen.

7. Pharmazeutische Präparate, dadurch gekennzeichnet, daß sie als wirksamen Bestandteil ein Derivat der allgemeinen Formel (I) gemäß einem der Ansprüche 1 und 2 enthalten.

**Ansprüche** (für den Vertragsstaat AT)

1. Verfahren zur Herstellung von Biphenyl-Alkyl-Carboxylat-derivaten mit der allgemeinen Formel (I) :

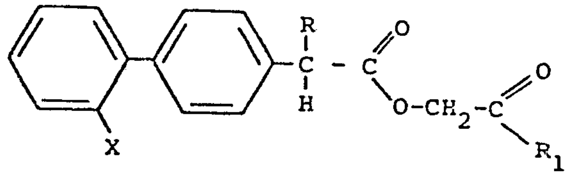

(I)

in welcher :

X ein Wasserstoff- oder Halogenatom

R ein Wasserstoffatom oder eine gerad- oder verzweigtkettige Alkylgruppe mit 1 bis 4 Kohlenstoffatomen,

$R_1$ die folgenden Gruppen darstellt :

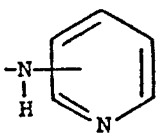 , 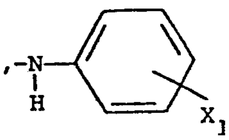 , 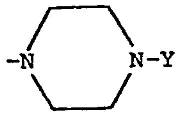 , 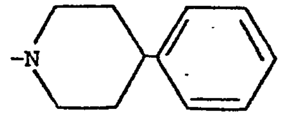

in welchen :

$X_1$ ein Wasserstoff- oder Halogenatom oder ein $CF_3$-Radikal,

Y eine gerad- oder verzweigtkettige Alkylgruppe mit 1 bis 4 Kohlenstoffatomen oder eine gegebenenfalls durch ein Halogenatom oder ein $CF_3$-Radikal substituierte Phenylgruppe darstellt,

sowie von deren therapeutisch annehmbaren Salzen, dadurch gekennzeichnet, daß eine Säure mit der Formel (II) :

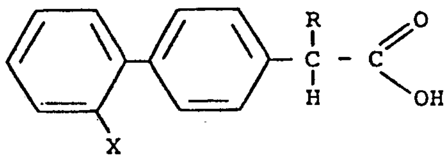

(II)

mit einem Chloracetylderivat der allgemeinen Formel (III) :

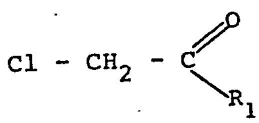

(III)

in Gegenwart von Alkalicarbonat und eines Katalysators behandelt wird, wobei X, R une $R_1$ die gleiche Bedeutung wie oben haben.

2. Verfahren nach Anspruch 1, dadurch gekennzeichnet, daß das Alkalicarbonat Kaliumcarbonat ist.

3. Verfahren nach einem der Ansprüche 1 und 2, dadurch gekennzeichnet, daß der Katalysator Kaliumjodid ist.

4. Verfahren nach einem der Ansprüche 1 bis 3, dadurch gekennzeichnet, daß ein aus den folgenden gewähltes Derivat hergestellt wird :

— N-Phenyl-para-biphenyl-acetoxyacetamid ;
— N(meta-Trifluoromethyl-phenyl)-para-biphenyl-acetoxy-acetamid ;
— Methyl-4-para-biphenyl-acetoxy-acetyl-1-piperazinchlorhydrat ;
— Phenyl-4-para-biphenyl-acetoxy-acetyl-1 piperidin ;
— (meta-Trifluormethyl-phenyl-4)-para-biphenyl-acetoxy-acetyl-1 piperazin-chlorhydrat ;
— Phenyl-4 [(phenyl-4 phenyl)-acetoxyacetyl]-1 piperazin ;
— Methyl-4 [(orthochlorphenyl-4 phenyl)-acetoxy-acetyl]-1 piperazin-chlorhydrat ;
— Phenyl-4 [(orthochlorphenyl)-4 phenylacetoxy-acetyl]-1-piperazin-chlorhydrat ;
— meta-Tri-fluormethyl-phenyl-4 [(orthochlorphenyl)-4-phenylacetoxy-acetyl]-1 piperazin-chlorhydrat ;
— (meta-Chlorphenyl)-4 [(orthochlorphenyl-4-phenyl)-acetoxy-acetyl]-1 piperazin-chlorhydrat ;
— [(Phenyl-4 piperazinyl-1)-2-oxo-2]-ethyl-para-biphenyl-2-propionat ;
— (Methyl-4 piperazino)-2-oxo-2 ethyl-chlor-2′-biphenylyl-4)-2 propionat-chlorhydrat ;
— (Phenyl-4 piperazino)-2 oxo-2-ethyl-chlor-2′ biphenylyl-4)-2 propionat ;
— (Phenyl-4 piperidino)-2 oxo-2-ethyl-chlor-2′-biphenylyl-4)-2 propionat ;
— (Amino-2-pyridin)-2 oxo-2 ethyl-oxalat-chlor-2′-biphenylyl-4)-2 propionat.